# EUROPEAN PATENT APPLICATION

(11) **EP 1 588 681 A1**
(43) Date of publication of application: **26.10.2005**
(21) Application number: 04706332.6
(22) Date of filing: 29.01.2004
(51) Int. Cl.: A61F 7/00, A61B 18/02

(54) **TEMPERATURE CONTROL DEVICE AND TEMPERATURE CONTROL METHOD BOTH USING PELTIER ELEMENT**

(30) Priority: 29.01.2003 JP 2003021193
(71) Applicant: Mayekawa Mfg. Co., Ltd., Tokyo 135-0046 (JP); Maruyama, Shigenao, Sendai-shi, Miyagi 9820801 (JP)
(72) Inventor: MARUYAMA, Shigenao, Sendai-shi, Miyagi 9820801 (JP); FUJIMA, Katsumi, c/o Mayekawa Mfg. Co., Ltd., Tokyo 1350046 (JP); YOSHIKAWA, Choiku, c/o Mayekawa Mfg. Co., Ltd., Tokyo 1350046 (JP); FUKANO, Shuji, c/o Mayekawa Mfg. Co., Ltd., Tokyo 1350046 (JP)
(74) Representative: Strehl, Peter
(86) International application number: PCT/JP2004/000842
(87) International publication number: WO 2004/066893

(57) **Abstract**

There are provided a device for controlling temperature by Peltier element and a method for controlling temperature by the same, wherein the structure of the device can be simplified, the cost making the device can be reduced and an accuracy of the temperature control of a contact plane based on the detected temperature value with a temperature sensor can be improved by capacitating insulation for a Peltier element and related elements without providing a vacuum case and related elements for encapsulating a Peltier element and related elements.

In a device for controlling temperature by Peltier element, wherein a temperature-controlled body is rapidly cooled or heated by Peltier effect generated by contacting the temperature-controlled body with the contact plane connected to a conductive body and by applying a direct current voltage between the Peltier element and the electrode-cum-heat-sink, a device for controlling temperature by Peltier element is **characterized in that** there are provided an end cover which covers the conductive body, the Peltier element and a whole of the electrode-cum-heat-sink or a part of the conductive body side and which forms a contact plane; a gas layer sealed in a space formed inside the cover; and an insulating layer comprising insulating material in the space formed inside the cover between the conductive body and the gas layer.

## Description

### Field of the Invention

The present invention relates to a device for controlling temperature and a method for controlling temperature by a Peltier element using a Peltier element that capacitates cooling with a large temperature difference and a big heat flux and that cools or heats rapidly a small local area, and more particularly an devicedevice for controlling temperature and a method for controlling temperature by a Peltier element appropriate for the local freezing treatment to dermatological lesions such as cutaneous or mucosal carsinoma.

### Description of the Related Art

Although electronic refrigerating systems using Peltier elements are applied to local cooling or freezing for optical instruments or electronic parts because a cooling temperature may control in accordance with a supplying electronic power, it is difficult to realize a large temperature difference and a big heat flux due to its low cooling performance at the steady state.

A module having multistage Peltier elements has been developed. On Japanese laid-open patent publication No. H8-186205, a temperature controlling device is disclosed wherein a heat sink contacted with a first Peltier element is further contacted with a second Peltier element. In such temperature controlling device, the second Peltier element needs to be larger than the first one in order to enhance the efficiency of the heat diffusion and heat transfer. That is, in case of the above mentioned multistage type device, the module next stage or after next stages needs to be larger than the one of the previous stage for enlarging the absorbing capacity to give temperature difference and to absorb heat evolved at the first stage so that the whole module becomes big and the control of cooling surfaces is apt to become unstable owing to the heat transfer and heat convection of the surrounding atmosphere.

Meanwhile, in the bloodless treatment for aged patients who cannot bear surgical abdominal operations or in the local treatment to dermatological lesions such as cutaneous or mucosal carsinoma, freezing operations wherein phenomena that arise with freezing is utilized for treatment by freezing the affected area of a living organism have been performed, in which liquid nitrogen has been conventionally used for freezing.

However, with the conventional method using liquid nitrogen, it is difficult to control the temperature appropriate for treating the tissue of living organism and over freezing by liquid nitrogen is apt to give damages to normal tissues adjacent to the freezing region, which lead to an unsatisfactory treatment.

As an art for overcoming the problem, a Peltier cautery instrument is disclosed on Japanese laid-open patent publication No. JP2002-177296.

The Peltier cautery instrument comprises a Peltier element having one of planes contacting a contact surface of a cauterized surface, that is a surface of a temperature-controlled body, an electrode-cum-heat-sink disposed on another of planes of the element so as to contact that plane, a temperature sensor disposed on the end or inner surface of the electrode-cum-heat-sink at the Peltier element side, an external cooler disposed on a back end of the electrode-cum-heat-sink, which does not contact the Peltier element, so as to contact that back end, wherein the plane of the Peltier element contacting a cauterized surface and the structure of the instrument except for the back end of the external cooler are sealed in a vacuum case.

When a cauterized surface or a surface of a temperature-controlled body is rapidly cooled with the Peltier cautery instrument, one of the planes disposed on the cauterized surface is a transient cooling plane of the Peltier element and the other of the planes in thermal contact with the heat sink member is a heating plane.

At a steady-state time, a temperature of the heat sink member is kept by the external cooler at a predetermined temperature lower than the temperature of the cauterize surface at a steady-state time and a predetermined temperature at a steady-state time is kept by applying the electric current to the Peltier element along such direction that the cauterized surface side is a heating plane. When rapid cooling, the applied current is inverted to a cooling mode from a heating mode so as to fulfill rapid cooling.

Though in the above referenced patent publication, an electronic refrigerating system having a large temperature difference and a big heat flux is realized utilizing a Peltier element, problems such as a large module and instability of temperature control remain, and the system lacks in practicality.

The liquid nitrogen treatment used for a medical refrigerating system such as a freezing operation has a difficulty of controlling temperature.

Though the art described in the above referenced patent publication can solve the above problems that the art described in the above referenced patent publication and the liquid nitrogen treatment have, the art described in the above referenced patent publication has following problems.
(1) Since the plane of the Peltier element contacting a cauterized surface, that is a surface of a temperature-controlled body, and the structure of the instrument except for the back end of the external cooler are sealed in a vacuum case so as to insulate heat, the instrument needs a device for generating and keeping vacuum such as a vacuum pump and a vacuum piping, whereby the cost to make the instrument increases because a structure of the temperature controlling instrument becomes complicated.
   A long operation of the instrument is apt to cause the reduction of the accuracy of temperature control due to lowering of the vacuum so that reliability as a temperature-controlling instrument for medical use is in question.
   Further, since, as described above, a main part of the Peltier element is installed in the vacuum case, a length of the conductive body having a contact plane to the surface of a temperature-controlled body is restricted, so that it is difficult to vary the shape of the end part of the temperature-control instrument as usage.
(2) Since, as described above, the Peltier element and the temperature-controlling element comprising the connecting element is sealed in a vacuum case, a temperature sensor for controlling temperature to monitor an operation is substantially impossible to dispose in the vicinity of the contact plane of the surface of a temperature-controlled body. The sensor is obliged to be disposed at the end or inner surface of the electrode-cum-heat-sink at the Peltier element side so that the temperature of contacting plane of the temperature- controlled surface cannot be detected accurately, which results in reduction of temperature controlling accuracy.

### SUMMARY OF THE INVENTION

In view of the abovementioned problems, the object of the present invention is to provide a temperature control device and method using a Peltier element, which enables to improve the accuracy of controlling temperature of a contacting plane based on the detected value of temperature with a temperature sensor and to reduce the cost to make the device by simplifying the structure of the device because of enabling insulation of a contacting element and the Peltier element without providing a vacuum case in which the Peltier element is sealed and related devices.

The present invention, in order to attain the above object, proposes an device for controlling temperature of a body to be controlled in temperature (hereafter referred to a temperature-controlled body) by contacting a surface of the device to said body, the device comprising a Peltier element consisting of a pair of semiconductors, an electrically conductive body contacting with an end surface of the Peltier element, an electrode-cum-heat-sink body contacting with the other end surface of the Peltier element, said electrically conductive body being able to be rapidly cooled or heated by virtue of Peltier effect generated by applying DC voltage between the Peltier element and the electrode-cum-heat-sink to cool or heat said temperature- controlled body contacted with said electrically conductive body, wherein an end cover made of electrical insulating material is provided to cover said electrically conductive body and Peltier element such that a closed space is formed around the electrically conductive body and Peltier element, or around the Peltier element, said end cover being formed to have an end plane part to be contacted with said temperature-controlled body when cooling or heating said temperature-controlled body, the inside surface of the end plane part of said end cover or whole or part of the inner peripheral part of said end cover being contacted with said electrically conductive body; and wherein a gas such as air, chlorofluorocarbon, argon, or others are sealed in said space to form heat insulating stratum.

In consequence of the above invention, since an end cover made of electrical insulating material is provided to cover said electrically conductive body and Peltier element such that a closed space is formed around the electrically conductive body and Peltier element, or around the Peltier element, said end cover being formed to have an end plane part to be contacted with said temperature-controlled body when cooling or heating said temperature-controlled body, the inside surface of the end plane part of said end cover or whole or part of the inner peripheral part of said end cover being contacted with said electrically conductive body and a gas such as air, chlorofluorocarbon, argon, or others are sealed in said space to form heat insulating stratum, the Peltier element and the electrode-cum-heat-sink is secured by the heat insulating stratum surrounded by the end cover.

Therefore, the intruded heat to the end part near the contact plane does not convect by the heat insulating stratum and is blocked to perform stable insulating action.

Since the vacuum case described in the above referenced patent publication is unnecessary, the conductive body can be made long and a form of the conductive body such as a diminution shape or an increasing-taper shape can be selected without restraint.

Therefore, according to the above invention, since temperature-controlling elements such as the conductive body, the Peltier element and the electrode-cum-heat-sink are securely insulated by the heat insulating stratum surrounded by the end cover, the device dose not needs a device for generating and keeping vacuum such as a vacuum pump and a vacuum piping that are needed for a conventional art so that the structure of the device can be simplified and the cost making the device can be reduced.

Further, as previously described, since the temperature-controlling elements are insulated by the heat insulating stratum surrounded by the end cover so that a device for generating and keeping vacuum including the vacuum case are unnecessary, the reduction of the accuracy of temperature control due to lowering of the vacuum is avoided even in case of a long operation so that reliability of the temperature-controlling device, especially as a temperature-controlling device for medical use, is improved.

According to the present invention, the end cover is shaped to be tapered towards its end plane part.

In this way, as the end part has a diminution shape, when a human body is treated, a contact area to the human body of the temperature-controlled body, that is a treating area, is well visible so as to be easy to treat. Further, as the end part becomes long, an insulating layer of the end part can be made long so that insulating effect for the temperature-controlling elements is enhanced.

Further, frost and dew formation in the vicinity of the contact plane is avoided by the heat insulating stratum surrounded by the end cover, which is formed with the outer shape of diminution by lengthening the conductive body.

According to the present invention, the electrically conductive body is of an inversed tapered shape enlarged towards its end side contacting with the end plane part of the end cover.

Consequently, as a contact area with the temperature-controlled body becomes large, a wide area of affected area is possible without enlarging the outer shape of the device for controlling temperature by Peltier element when treating a human body.

Further according to the present invention, the electrically conductive body is formed into a shape bowing along length and reducing in girth or diameter towards its end side contacting with the end plane part of the end cover.

Thus, as the contact plane side of the diminution-shaped end part is bent, treatment for a narrow part such as an oral inside becomes easy.

The present invention proposes a method of controlling temperature of a body by an device for controlling temperature of a body to be controlled in temperature(hereafter referred to a temperature-controlled body) by contacting a surface of the device to said body, the device comprising a Peltier element consisting of a pair of semiconductors, an electrically conductive body contacting with an end surface of the Peltier element, an electrode-cum-heat-sink body contacting with the other end surface of the Peltier element, said electrically conductive body being able to be rapidly cooled or heated by virtue of Peltier effect generated by applying DC voltage between the Peltier element and the electrode-cum-heat-sink to cool or heat said temperature-controlled body contacted with said electrically conductive body, said surface of the device being formed by an end plane part of an end cover made of electrical insulating material, said method comprising: detecting temperature of the end plane part of the end cover being contacted with said temperature-controlled body by a temperature sensor; controlling temperature of the end plane part of the end cover being contacted with said temperature-controlled body based on the detected temperature by adjusting voltage applied to said Peltier element.

Further according to the present invention, a temperature sensor is provided on the inside surface of the end plane part of said end cover to detect the temperature of the end plane part of the end cover.

Further according to the present invention, a plurality of said electrically conductive bodies each varying in its area of the side contacting to the inside surface of the end plane part of the end cover are prepared, and one of said conductive bodies is adopted in accordance with the area of the temperature-controlled body to be contacted with the surface of the device.

Thus, since a vacuum case for encapsulating the temperature control element is unnecessary because the insulation of the temperature control element is assured with the heat insulating stratum surrounded by the end cover, the temperature sensor for controlling a temperature at the time of monitoring an operation is easily disposed in the vicinity of the contact plane of a temperature-controlled body so that the temperature of the contact plane of the temperature-controlled plane is precisely detected, whereby monitoring an operation based on the detected temperature of the contact plane can be precisely performed.

Further, when a plurality of electrically conductive bodies each varying in its area of the side contacting to the inside surface of the end plane part of the end cover are prepared, one of said conductive bodies is adopted to use corresponding to the state of an affected area of a human body.

Further, when the temperature-controlled body is an affected area of a human body, it is preferable that a time when the temperature detected by said temperature sensor reaches a temperature to cancel overcooling is detected, and timing to start operation for the affected area is determined based on said detected time to cancel overcooling.

Thus, the starting time of freezing an affected area of a human body can be precisely found by detecting a temperature to cancel over cooling with the temperature sensor disposed in the vicinity of the affected area of a human body whereby timing to start operation for the affected area is determined based on said detected time to cancel overcooling and operation can be performed while monitoring an operation time and the depth of an operating section.

Further, according to the present invention, voltage applied to the Peltier element and time for applying the voltage are adjusted based on the temperature of the end plane part of the end cover detected by said temperature sensor and a contact area of the end plate part of the end cover with the temperature-controlled body.
Thus, applying voltage to the Peltier element is reversed from heating mode to cooling mode or vice versa so as to change rapid cooling to rapid heating or vice versa corresponding to the state of the operating section, or an extent or a time of duration of applying voltage to the Peltier element is adjusted so as to hold a temperature and a duration time for warming most appropriate for the state of the operating section.

### BRIEF DESCRIPTION OF THE DRAWINGS

Fig. 1 is a sectional view of a device for controlling temperature by Peltier element with regard to a first embodiment of the present invention.
Fig. 2 is a sectional view of the end part of n device for controlling temperature by Peltier element with regard to a second embodiment of the present invention.
Fig. 3 is a sectional view of the end part of a device for controlling temperature by Peltier element with regard to a third embodiment of the present invention.
Fig. 4 is a graph showing a temperature variation at a time of operation using the devices for controlling temperature with regard to the above embodiments.

### DESCRIPTION OF THE PREFERRED EMBODIMENT

The invention will now be described in detail by way of example with reference to the accompanying drawings. It should be understood, however, that the description herein of specific embodiments such as to the dimensions, the kinds of material, the configurations and the relative disposals of the elemental parts and the like is not intended to limit the invention to the particular forms disclosed but the intention is to disclose for the sake of example unless otherwise specifically described.

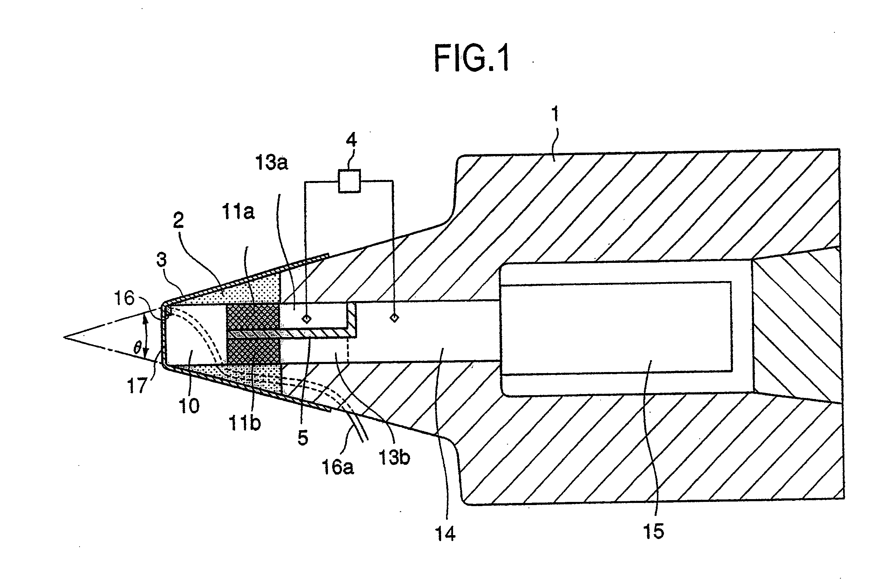

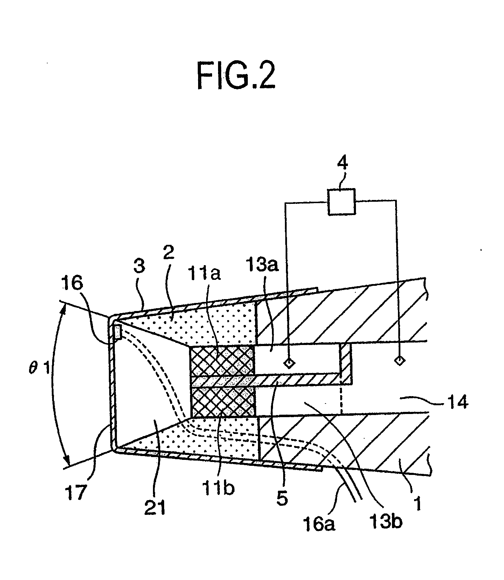

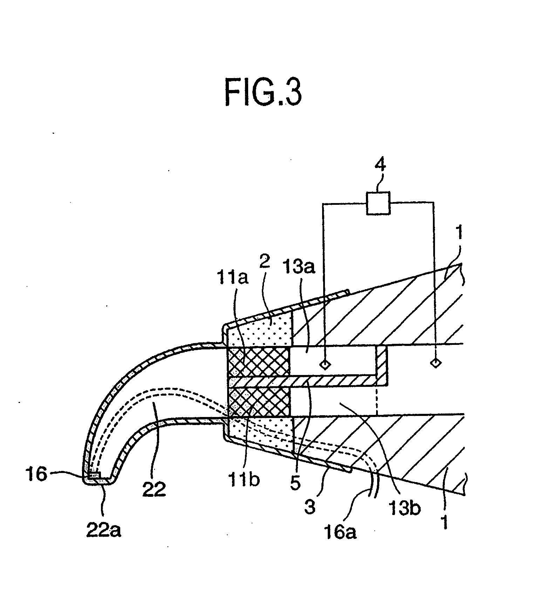

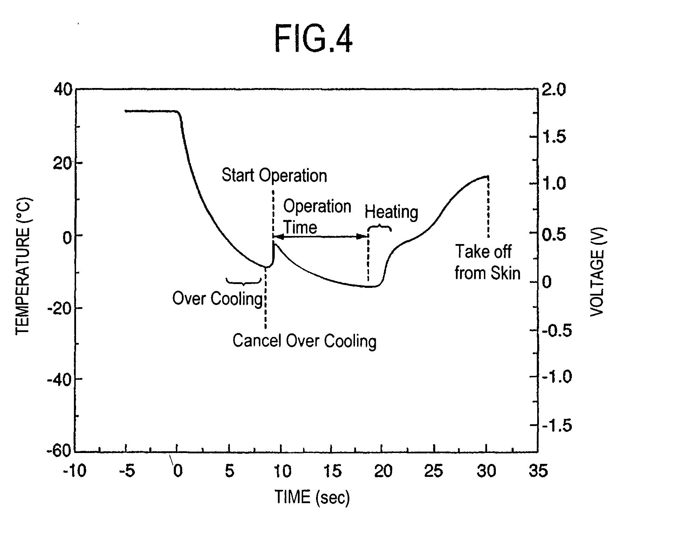

Fig. 1 is a sectional view of a device for controlling temperature by Peltier element with regard to a first embodiment of the present invention; Fig. 2 is a sectional view of the end part of a device for controlling temperature by Peltier element with regard to a second embodiment of the present invention; and Fig. 3 is a sectional view of the end part of a device for controlling temperature by Peltier element with regard to a third embodiment of the present invention. Fig. 4 is a graph showing a temperature variation at a time of operation using the devices for controlling temperature with regard to the above embodiments.

In Fig. 1 (A) in which a first embodiment is shown, a Peltier element used in the present invention comprises a p-type circuit wherein heat semiconductors comprising a general P-type semiconductor **11a** and a N-type semiconductor **11b** are connected with a metal conductor **10.**

Though the P-type semiconductor **11a** and N-type semiconductor **11b** are usually disposed with a slight distance therebetween, they are disposed through the intermediary of an electrically insulating layer **5** so as to achieve a compact structure according to the device for controlling temperature of the present invention.

**10** is a metal conductor which adheres to the end part surfaces of the P-type element and N-type element of the Peltier element. The end plane of the metal conductor is disposed inside a contact plane **17** for contacting a temperature-controlled body (a detail of which is given afterward) such as a suffered area of a human body.

Each of electrodes **13a, 13b** is disposed so as to contact the end plane of the P-type semiconductor **11a** and the N-type semiconductor **11b** of the p-type circuit, which is the opposite side plane to the metal conductor **10.**

**14** is a heat sink which eradiates the heat generated at the one side of the Peltier elements **11a, 11b,** which is the side opposite to the metal conductor **10.** The heat sink **14** is contacted in such a manner that it forms one body with one of the electrodes **13a, 13b** which is disposed to be contacted with each of the P-type element **11a** or the N-type element **11b** of the Peltier element and is disposed to contact another electrode through the intermediary of the electrically insulating layer **5,** thereby forms an electrode-cum-hest-sink.

Since the heat sink **14** is an electrode-cum-hest-sink, a material having both excellent heat and electric conductivity such as copper or aluminum is used.

**4** is an electric leading circuit. The contact plane **17** of the metal conductor **10** with a temperature-controlled body such as an affected area of a human body is cooled or heated by applying a direct current voltage between the electrode **13a** of the p-type circuit and the electrode-cum-hest-sink **14** with the electric leading circuit **4.**

An external cooler **15** having a structure capable of containing cooling agent such as dry ice is disposed to contact with a rear end plane of the heat sink **14,** which does not contact with the Peltier element **11a, 11b.**

Encapsulating cooling agent in the external cooler **15** attains rapid cooling of the end of the device because of rapid removal of heat.

Further, if necessary, a circulating tubing part of a circulating cooling device which is disposed differently may be connected to the external cooler **15** for cooling.

**1** is a heat insulator comprising solid heat insulating material. It insulates heat by covering the root part of the temperature controlling element comprising the metal conductor **10,** the Peltier elements **11a, 11b,** the electrodes **13a, 13b** and the heat sink **14,** that is, the outer circumference of the opposite side part to the metal conductor **10** and the outer circumference of the external cooler **15.**

**3** is an end cover comprising electrical insulating material (preferably translucent in color) such as hard synthetic resin. The end part thereof is formed to be a thin cylinder the end part of which becomes reduced to the end in its sectional area and a contact plane **17** contacting the temperature-controlled body is formed at the end.

An outer shape including the end cover is formed as a diminution shape in which the end part becomes reduced to the end in its sectional area. The end part of the temperature-controlling element comprising the metal conductor **10,** the Peltier elements **11a, 11b,** the electrodes **13a, 13b** and the heat sink **14** is covered, while the root part is fluid-tightly fixed to the end part of the heat insulator. A point angle of the part provided with the end cover **3** is preferably from **30** to **45** degrees.

In the sealed space formed between the inside of the cover **3** of the end part and the outer circumference of the temperature controlling element comprising the metal conductor **10** covered with the end cover **3,** the Peltier elements **11a, 11b,** the electrodes **13a, 13b** and the heat sink **14,** a heat insulating stratum **2** such as air, chlorofluorocarbon, argon, and xenon is provided. Therefore, the end part of the temperature controlling element comprising the metal conductor **10,** the Peltier elements **11a, 11b,** the electrodes **13a, 13b** and the heat sink **14** is surly insulated with the heat insulating stratum **2** in the sealed space sealed by the end cover **3** so that the temperature controlling element can be insulated without using such vacuum case as described in Japanese laid-open patent No. P2002-177296.

**16** is a temperature sensor provided on the metal conductor **10.** Though a known temperature sensor may be used such as a thermocouple and a thermistor, the temperature sensor **16** is disposed at the nearest position to the contact plane **17** of the metal conductor **10** in order to detect accurately the temperature of the contact plane **17** of the metal conductor that is a point a temperature of which is controlled. In this embodiment, the sensor is disposed at the end point surface of the outer circumference of the metal conductor **10.** A signal of the detected value from the temperature sensor **16** is communicated to a temperature controlling device through a detecting line **16a.**

A method for controlling temperature using the device for controlling temperature by Peltier element thus constructed as described above is explained as follows.

A signal of the temperature value detected by the temperature sensor **16** is constantly input to a controller through the detecting line **16a.** The controller may be provided separately from the device for controlling temperature shown in Fig. 1 and connected to the electrodes **13a, 13b** of the Peltier elements **11a, 11b** with a lead wire. The controller is not particularly restricted so long as it is capable of varying a state of applying electric current, that is, an applied voltage to the Peltier elements **11a, 11b** based on the temperature signal.

In the controller, in case of operation of a human body, as shown in Fig. 4, an hourly standard temperature for operation (targeted temperature) and a standard applied voltage corresponding to the standard temperature at an affected area of operating object which contacts the contact plane **17** of the metal conductor **10** are set. An applied voltage is adjusted so that a temperature of the contact plane **17** coincides with the standard temperature by comparing a constantly detected value of temperature from the temperature sensor with the standard temperature. Thus, a temperature of the contact plane 17 that contacts an affected area of operating object is always adjusted to the standard temperature (targeted temperature).

Therefore, as the temperature-controlling element is surly insulated by the heat insulating stratum **2** surrounded by the end cover **3,** a vacuum case for encapsulating the temperature-controlling element is unnecessary so that a temperature of the contact plane **17** of an affected area of a human body is precisely detected by disposing the temperature sensor **16** for temperature control at monitoring operation. Thus, monitoring operation based on a detected value of a contact temperature from the temperature sensor **16** can be accurately performed.

In such embodiment, in case of operation of a human body, as shown in Fig. 4, a temperature for canceling over cooling is detected with the temperature sensor **16** disposed on the metal conductor **10** in the vicinity of the contact plane 17, and start to operate the affected area is determined from the temperature thereof. Thus, the starting time of freezing an affected area of a human body can be precisely found by detecting a temperature for canceling over cooling with the temperature sensor **16** disposed in the vicinity of the affected area of a human body whereby a starting time for operating the affected area is determined from the temperature thereof and operation can be performed while monitoring an operation time and the depth of an operating section.

In such embodiment, a voltage applied to the Peltier elements **11a, 11b** and a time for applying voltage thereto are preferably adjusted corresponding to a temperature detected with the temperature sensor **16** in the vicinity of the contact plane and to a contacting area of the affected section of a human body (temperature-controlled body).

Thus, applying voltage to the Peltier element **11a, 11b** is reversed from heating mode to cooling mode or vice versa so as to change rapid cooling to rapid heating or vice versa corresponding to the state of the operating section, or an extent or a time of duration of applying voltage to the Peltier element **11a, 11b** is adjusted so as to hold a temperature and a duration time for warming most appropriate for the state of the operating section.

As described above, according to such embodiment, since the conductive body **10,** the Peltier elements **11a, 11b** and a whole of the electrode-cum-heat-sink **14** or a part of the contact plane **17** side to the temperature-controlled body (affected section) are covered with an end cover **3** and a heat insulating stratum **2** is formed by sealing air, chlorofluorocarbon, argon or others in a space formed inside the cover **3,** the insulation of the conductive body 10, the Peltier elements **11a, 11b** and the electrode-cum-heat-sink **14** is secured by the heat insulating stratum 2 surrounded by the end cover **3.**

Further, the intruded heat to the end part near the contact plane 17 does not convect by the heat insulating stratum and is blocked to perform stable insulating action.

Therefore, the device dose not need a device for generating and keeping vacuum such as a vacuum pump and a vacuum piping for encapsulating the metal conductor **10,** Peltier elements **11a, 11b** and the electrode-cum-heat-sink **14** that are needed for a conventional art.

Further, since the temperature-controlling elements are insulated by the heat insulating stratum **2** surrounded by the end cover 3 so that a device for generating and keeping vacuum including the vacuum case are unnecessary, the reduction of the accuracy of temperature control due to lowering of the vacuum is avoided even in case of a long operation so that reliability of the temperature-controlling device, especially as a temperature-controlling device for medical use, is improved.

Further, in such embodiment, since an outer shape including the end cover **3** is formed as a diminution shape in which the end part becomes reduced to the end in its sectional area, when a human body is treated, a contact area to the human body of the temperature-controlled body, that is a treating area, is well visible so as to be easy to treat. Further, as the end part becomes long, an insulating layer of the end part can be made long so that insulating effect for the temperature-controlling elements is enhanced.

In a second embodiment shown in Fig. 2, the metal conductor **21** is formed as an expanding-taper shape having an expanding taper angle θ₁ in which contact plane **17** side to an affected section (a temperature-controlled body) expands. The end cover is also formed corresponding to the shape of the metal conductor 21. According to this embodiment, as a contact area of the contact plane to contact an affected section of a human body becomes large, a wide area of an affected section can be treated without enlarging the outer shape of the device for controlling temperature when a human body is treated. Other structures are the same as the first embodiment and the same sign denotes the same member.

In a third embodiment shown in Fig. 3, the conductive body is formed in such a shape that the contact plane side is reduced and bent to a predetermined shape. According to the third embodiment, as the contact plane side of the diminution-shaped end part is bent, treatment for a narrow part such as an oral inside becomes easy. If the metal conductors and the end covers having different bent forms are prepared, the metal conductor and the end cover can be selected and changed to use corresponding to the state of the affected section. Other structures are the same as the first embodiment and the same sign denotes the same member.

### INDUSTRIAL APPLICABILITY

As described above, according to the present invention, since temperature-controlling elements such as the conductive body, the Peltier element and the electrode-cum-heat-sink are securely insulated by the heat insulating stratum surrounded by the end cover, the device dose not needs a device for generating and keeping vacuum such as a vacuum pump and a vacuum piping that are needed for a conventional art so that the structure of the device can be simplified and the cost making the device can be reduced.

Further, since a vacuum case that is needed for a conventional art is unnecessary, a length of the conductor is made long so as to be capable of make the shape as a diminution shape or an expanding taper shape, whereby the shape can be widely selected as usage.

Further, since the temperature-controlling elements are insulated by the heat insulating stratum surrounded by the end cover so that a device for generating and keeping vacuum including the vacuum case are unnecessary, the reduction of the accuracy of temperature control due to lowering of the vacuum is avoided even in case of a long operation so that reliability of the temperature-controlling device, especially as a temperature-controlling device for medical use, is improved.

Therefore, as the temperature-controlling element is surly insulated by the heat insulating stratum surrounded by the end cover, a vacuum case for encapsulating the temperature-controlling element is unnecessary so that a temperature of the contact plane of an affected area of a human body is precisely detected by disposing the temperature sensor for temperature control at monitoring operation. Thus, monitoring operation based on a detected value of a contact temperature from the temperature sensor can be accurately performed.

## Claims

1. A device for controlling temperature of a body to be controlled in temperature(hererafter referred to a temperature-controlled body) by contacting a surface of the device to said body, the device comprising a Peltier element consisting of a pair of semiconductors, an electrically conductive body contacting with an end surface of the Peltier element, an electrode-cum-heat-sink body contacting with the other end surface of the Peltier element, said electrically conductive body being able to be rapidly cooled or heated by virtue of Peltier effect generated by applying DC voltage between the Peltier element and the electrode-cum-heat-sink to cool or heat said temperature- controlled body contacted with said electrically conductive body,
wherein an end cover made of electrical insulating material is provided to cover said electrically conductive body and Peltier element such that a closed space is formed around the electrically conductive body and Peltier element, or around the Peltier element, said end cover being formed to have an end plane part to be contacted with said temperature-controlled body when cooling or heating said temperature-controlled body, the inside surface of the end plane part of said end cover or whole or part of the inner peripheral part of said end cover being contacted with said electrically conductive body; and
wherein a gas such as air, chlorofluorocarbon, argon, or others are sealed in said space to form heat insulating stratum.

2. A device according to claim 1, wherein a temperature sensor is provided on the inner surface of the end plane part of said end cover to detect the temperature of the end plane part of the end cover.

3. A device according to claim 1, wherein said end cover is shaped to be tapered towards its end plane part.

4. A device according to claim 1, wherein said electrically conductive body is of an inversed tapered shape enlarged towards its end side contacting with the end plane part of the end cover.

5. A device according to claim 1, wherein said electrically conductive body is formed into a shape bowing along length and reducing in girth or diameter towards its end side contacting with the end plane part of the end cover.

6. A method of controlling temperature of a body by an device for controlling temperature of a body to be controlled in temperature(hererafter referred to a temperature-controlled body) by contacting a surface of the device to said body, the device comprising a Peltier element consisting of a pair of semiconductors, an electrically conductive body contacting with an end surface of the Peltier element, an electrode-cum-heat-sink body contacting with the other end surface of the Peltier element, said electrically conductive body being able to be rapidly cooled or heated by virtue of Peltier effect generated by applying d.c. voltage between the Peltier element and the electrode-cum-heat-sink to cool or heat said temperature- controlled body contacted with said electrically conductive body, said surface of the device being formed by an end plane part of an end cover made of electrical insulating material, said method comprising:
detecting temperature of the end plane part of the end cover being contacted with said temperature-controlled body by a temperature sensor;
controlling temperature of the end plane part of the end cover being contacted with said temperature-controlled body based on the detected temperature by adjusting voltage applied to said Peltier element.

7. A method of controlling temperature of a body according to claim 6, wherein voltage applied to the peltier element and time for applying the voltage are adjusted based on the temperature of the end plane part of the end cover detected by said temperature sensor and a contact area of the end plate part of the end cover with the temperature-controlled body.

8. A method of controlling temperature of a body according to claim 6, wherein said temperature-controlled body is an affected area of a human body, the method comprising:detecting a time when the temperature detected by said temperature sensor reaches a temperature to cancel overcooling, determining timing to start operation for the affected area based on said detected time to cancel overcooling.

9. A method of controlling temperature of a body according to claim 6, wherein a plurality of said electrically conductive bodies each varying in its area of the side contacting to the inside surface of the end plane part of the end cover are prepared, and one of said conductive bodies is adopted in accordance with the area of the temperature-controlled body to be contacted with the surface of the device.
